# EUROPÄISCHE PATENTSCHRIFT

(11) **EP 3 107 891 B1**
(45) Veröffentlichungstag und Bekanntmachung des Hinweises auf die Patenterteilung: **26.09.2018**
(21) Anmeldenummer: 15702456.3
(22) Anmeldetag: 02.02.2015
(51) Int. Cl.: C07C 209/26, C07C 211/29

(54) **VERFAHREN ZUR REDUKTIVEN AMINIERUNG VON ALDEHYDEN**
METHOD FOR THE REDUCTIVE AMINATION OF ALDEHYDES
PROCÉDÉ D'AMINATION RÉDUCTRICE D'ALDÉHYDES

(30) Priorität: 19.02.2014 EP 14155796
(43) Veröffentlichungstag der Anmeldung: 28.12.2016
(73) Patentinhaber: BASF SE, 67056 Ludwigshafen am Rhein (DE)
(72) Erfinder: EIDAMSHAUS, Christian, 68168 Wiesloch (DE); ALTENHOFF, Ansgar Gereon, 69120 Heidelberg (DE)
(74) Vertreter: BASF IP Association
(86) Internationale Anmeldenummer: PCT/EP2015/052028
(87) Internationale Veröffentlichungsnummer: WO 2015/124413

(56) Entgegenhaltungen:
- EP-A2- 0 816 323
- US-A1- 2002 137 970
- US-A1- 2007 232 833
- US-A1- 2013 116 451
- BHATTACHARYYA SUKANTA: "A high throughput synthesis of N,N-dimethyl tertiary amines", SYNTHETIC COMMUNICATIONS: AN INTERNATIONAL JOURNAL FOR RAPID COMMUNICATION OF SYNTHETIC ORGANIC CHEMISTRY, TAYLOR & FRANCIS INC, PHILADELPHIA, PA; US, Bd. 30, Nr. 11, 1. Januar 2000 (2000-01-01), Seiten 2001-2008, XP002698158, ISSN: 0039-7911

## Beschreibung

Die Erfindung betrifft Verfahren zur reduktiven Aminierung durch Umsetzung einer Carbonylverbindung mit einem sekundären Amin und Wasserstoff, welches dadurch gekennzeichnet ist, dass die Umsetzung kontinuierlich durchgeführt wird und das Reaktionsgemisch, welches die Carbonylverbindung, das sekundäre Amin und Wasserstoff enthält, zunächst mit einem sauren Festbett und danach mit einem Festbett des Hydrierkatalysator (im Nachfolgenden Hydrierfestbett genannt) in Kontakt gebracht wird.

Ein übliches und wirtschaftlich bedeutendes Verfahren zur Herstellung von Aminen ist die reduktive Aminierung. Bei einer reduktiven Aminierung werden Ketone oder Aldehyde zu einem Amin umgesetzt. Ein bekanntes Verfahren der reduktiven Aminierung ist die Umsetzung einer Carbonylverbindung mit einem Amin in Gegenwart von Wasserstoff, wie es z.B. in WO 01/05741 beschrieben ist.

In der früheren, nicht am Anmeldetag dieser Anmeldung noch nicht veröffentlichten europäischen Anmeldung (Aktenzeichen 13173233.1, BASF Zeichen PF 75487) (Publikation WO2014202346 A1) wird die Herstellung von 2-Chlordimethylbenzylamin durch reduktive Aminierung, das heißt durch Umsetzung von 2-Chlorbenzaldehyd mit einem Amin und Wasserstoff beschrieben.

Die Herstellung von 2-Chlordimethylbenzylamin wird von S. Bhattacharyya in Synth. Comm. 2000, vol 30 (11), Seiten 2001-2008, offenbart. Aufgabe der vorliegenden Erfindung war, das Verfahren der reduktiven Aminierung weiter zu verbessern. Gewünscht ist ein möglichst einfaches und wirtschaftliches Verfahren, welches kontinuierlich durchgeführt werden kann. Die Ausbeute an hergestellten Aminen und die Selektivität sollen möglichst hoch sein.

Demgemäß wurde das eingangs beschriebene Verfahren gefunden.

### Zu den Ausgangsstoffen

Es handelt sich um ein Verfahren, bei dem eine Carbonylverbindung, ein sekundäres Amin und Wasserstoff wie in den Ansprüchen definiert miteinander umgesetzt werden.

Eine Carbonylverbindung ist eine organische Verbindung mit mindestens einer Carbonylgruppe. Die Carbonylverbindung ist der 2-Chlorbenzaldehyd der Formel I 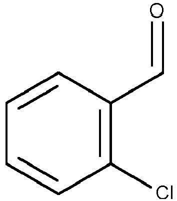 Das sekundäres Amin ist in den Ansprüchen definiert.

Bevorzugte sekundäre Amine haben ein Molgewicht von maximal 1000 g/mol, insbesondere maximal 500 g/mol, besonders bevorzugt maximal 300 g/mol. In einer ganz besonders bevorzugten Ausführungsform wird ein sekundäres Amin mit einem Molgewicht von maximal 200 g/mol eingesetzt.

Das sekundäre Amin ist vorzugsweise bei Normalbedingungen (20°C, 1 bar) flüssig oder gasförmig. In einer besonderen Ausführungsform ist es bei Normalbedingungen gasförmig. Das sekundäres Amin ist Formel II 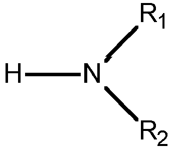 wobei R1 und R2 jeweils unabhängig voneinander für eine C1- bis C10 Alkylgruppe stehen. Vorzugsweise stehen R1 und R2 unabhängig voneinander für eine C1- bis C4-Alkylgruppe. Besonders bevorzugt stehen R1 und R2 jeweils für eine Methylgruppe. Durch das Verfahren werden 2-Chlordialkylbenzylamine der Formel III 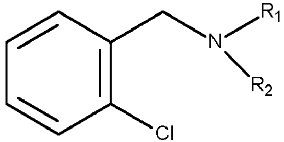 hergestellt.

Dazu werden ein Aldehyd der Formel I, ein Dialkylamin der Formel II und Wasserstoff als Ausgangsverbindungen eingesetzt.

Besonderes bevorzugt wird durch das Verfahren 2-Chlordimethylbenzylamin (R₁ und R₂ in Formel II und III stehen dann jeweils für eine Methylgruppe) hergestellt.

Bei der Umsetzung reagieren 2-Chlorbenzaldehyd und Dialkylamin voraussichtlich zu einem Zwischenprodukt, wobei es z. B. um das entsprechende Aminal, Halbaminal oder Iminium-Ion handeln kann. Dieses Zwischenprodukt wird in Gegenwart von Wasserstoff zum 2-Chlordialkylbenzylamin hydriert.

### Zur Umsetzung

Die Carbonylverbindung wird mit dem Dialkylamin und Wasserstoff umgesetzt.

Die Umsetzung kann ohne Mitverwendung eines Lösemittels oder unter Verwendung eines Lösemittels durchgeführt werden. Die Umsetzung kann z. B. in Gegenwart eines Lösemittels für die Carbonylverbindung oder für das Dialkylamin durchgeführt werden. Als Lösemittel für die Carbonylverbindung, insbesondere für 2-Chlorbenzaldehyd, geeignet sind z. B. hydrophile, organische Lösemittel. Besonders bevorzugt sind hydrophile, nicht protische, organische Lösemittel. Genannt seien z.B. Ether, wie Tetrahydrofuran, Diethylether, Methyltertiärbutylether oder 1,4-Dioxan. Als Lösemittel für das Dialkylamin kommen Wasser oder hydrophile organische Lösemittel, in denen das Dialkylamin zumindest teilweise löslich ist, in Betracht. Genannt seien insbesondere Wasser, Ether, wie Tetrahydrofuran oder Alkohole wie Methanol oder Ethanol.

Vorzugsweise wird bei der Umsetzung kein Lösemittel mitverwendet.

Die Carbonylverbindung, das Dialkylamin, der Wasserstoff und, falls mitverwendet, ein Lösemittel können dem Reaktor getrennt oder bereits als Mischung von mindestens zwei der genannten Ausgangsstoffe kontinuierlich zugeführt werden. In dem Rohreaktor erfolgt dann die Durchmischung der Ausgangsstoffe zum Reaktionsgemisch.

Insbesondere flüssige Ausgangsstoffe können vorab gemischt und dem Reaktor als flüssige Mischung zugeführt werden.

Bei dem Reaktor handelt es sich vorzugsweise um einen Rohrreaktor.

Wasserstoff wird gasförmig, vorzugsweise durch Einstellen und Aufrechterhaltung eines entsprechenden Wasserstoffdruckes, zugeführt. Wasserstoff kann gegebenenfalls auch im Gemisch mit Inertgasen wie Stickstoff oder Edelgase verwendet werden.

Gasförmige Dialkylamine können dem Reaktor zusammen mit dem Wasserstoffgas zugeführt werden. Dimethylamin hat z. B. einen Siedepunkt von 7°C. Der Wasserstoffstrom kann vorab mit dem Dimethylamin in Kontakt gebracht werden, so dass der Gasstrom ausreichend Dimethylamin enthält.

Vorzugsweise wird das Dialkylamin in mindestens equimolaren Mengen eingesetzt, insbesondere wird es in molarem Überschuss bezogen auf die Carbonylverbindung eingesetzt.

Insbesondere beträgt das Molverhältnis von Dialkylamin zur Carbonylverbindung 1 : 1 bis 50 : 1, besonders bevorzugt 2 : 1 bis 20 : 1 und ganz besonders bevorzugt 3 : 1 bis 15 : 1.

Der Wasserstoff wird gasförmig in ausreichenden Mengen, im Allgemeinen im molaren Überschuss, bezogen auf die Carbonylverbindung, zugeführt.

Die Umsetzung wird kontinuierlich durchgeführt, das heißt, dass die Ausgangsstoffe kontinuierlich zugeführt und die erhaltenen Produkte kontinuierlich entfernt werden.

Das Reaktionsgemisch, welches die Carbonylverbindung, das sekundäre Amin und Wasserstoff enthält, wird in dem Reaktor, vorzugsweise Rohrreaktor, zunächst mit einem sauren Festbett und danach mit einem Festbett des Hydrierkatalysator (im Nachfolgenden Hydrierfestbett genannt) in Kontakt gebracht.

Bei dem sauren Festbett handelt es sich um ein Festbett von sauren Feststoffteilchen.

Unter sauren Feststoffteilchen werden Feststoffteilchen verstanden, die insgesamt als Säure wirken. Daher enthalten die sauren Feststoffteilchen saure Verbindungen in einer Menge, dass die Feststoffteilchen insgesamt die Wirkung als Säure haben. Insbesondere handelt es sich um Feststoffteilchen, welche bei Zugabe von mindestens 10 g Feststoffteilchen in 100 g neutrales Wasser (pH 7) ein Absenkung des pH-Wertes bewirken, unabhängig davon, ob die Feststoffteilchen in Wasser löslich, teilweise löslich oder unlöslich sind. Für die Charakterisierung der sauren Eigenschaften können auch andere klassische Methoden herangezogen werden; dazu gehören Titrationsmethoden (z.B. mit Hammett-Indikatoren), Adsorptionsmethoden, spektroskopische Methoden und Testreaktionen.
Das saure Festbett enthält die sauren Feststoffteilchen oder besteht daraus. Neben den sauren Feststoffteilchen kann das saure Festbett weitere Bestandteile enthalten, zum Beispiel Inertmaterialen wie Füllstoffe.

Vorzugsweise enthält das saure Festbett Hydrierkatalysatoren nur in geringen Mengen, insbesondere enthält das saure Festbett keine Hydrierkatalysatoren. Das saure Festbett enthält insbesondere weniger als 10 Gew. %, besonders bevorzugt weniger als 5 Gew. %, ganz besonderes bevorzugt weniger als 1 Gew.% Hydrierkatalysatoren, bezogen auf das Gesamtgewicht des sauren Festbetts. Bei den Hydrierkatalysatoren handelt es sich insbesondere um solche wie sie weiter unten definiert und aufgelistet sind.

In einer bevorzugten Ausführungsform besteht das saure Festbett zu mindestens 50 Gew. %, insbesondere zu mindestens 80 Gew. %, besonderes bevorzugt zu mindestens 95 Gew % aus den sauren Feststoffteilchen. In einer besonderen Ausführungsform besteht das saure Festbett zu 100 Gew. % aus den sauren Feststoffteilchen.

Die sauren Feststoffteilchen können eine beliebige Form haben. Sie können als Pulver oder Körner vorliegen, sie können aber auch in eine beliebige andere Form gebracht werden, z. B. durch entsprechendes Verpressen. Geeignet sind Feststoffteilchen in der Form von Zylindern, Perlen, Ringen oder Tabletten. Die Feststoffteilchen können z.B. einen längsten Durchmesser von bis zu 50 mm haben.

Die sauren Feststoffteilchen bestehen vorzugsweise zu mindestens 20 Gew. %, insbesondere zu mindestens 50 Gew. % besonders bevorzugt zu mindestens 80 Gew. % aus sauren Verbindungen. Neben sauren Verbindungen können die Feststoffteilchen weitere Verbindungen, z. B. nicht saure Verbindungen als Träger, enthalten.
In einer besonders bevorzugten Ausführungsform bestehen die sauren Feststoffteilchen ausschließlich aus sauren Verbindungen.

Bei den sauren Verbindungen kann es sich Lewis-Säuren oder Brönstedtsäuren handeln. Bevorzugte saure Verbindungen sind saure Metalloxide, Phosphate, Wolframate, Sulfate und organische Säuren oder deren Salze.

Als saure Metalloxide seien insbesondere Titandioxid, Zirkoniumdioxid, Aluminiumoxid, Siliciumdioxid, oder Mischoxide von Aluminium und Silicium (Zeolithe) und saure Tonerden genannt. In Zeolithen können Aluminium- und Siliciumatome auch teilweise durch andere Atome ersetzt sein, z. B. kann Aluminium durch andere dreiwertige Metalle ersetzt sein.

Als saure organische Verbindungen kommen insbesondere organische lonenaustauscherharze mit Säuregruppen, zum Beispiel Carbonsäuregruppen oder Sulfonsäuregruppen, in Betracht. Zum Beispiel handelt es sich dabei um sulfonierte Copolymerisate aus Styrol und Divenylbenzol (z.B. der Marken Lewatit der Fa. Lanxess, Amberlite der Fa. Rohm & Haas).

In einer besonders bevorzugten Ausführungsform enthält das saure Festbett Metalloxide, insbesondere Zirkoniumdioxid, als saure Verbindung. In einer ganz besonders bevorzugten Ausführungsform besteht das saure Festbett ausschließlich aus Metalloxiden, insbesondere Zirkoniumdioxid.

Das saure Festbett kann in beliebiger Weise in den Reaktor eingebracht werden. Im Allgemeinen wird es so eingebracht, dass der gesamte Strom der Edukte durch das saure Festbett geführt wird. Das saure Festbett füllt daher im Allgemeinen den gesamten durchströmten Querschnitt des Reaktors aus. Das Volumen des sauren Festbettes wird vorzugsweise so gewählt, dass die Belastung des sauren Festbettes mit dem Massestrom der Carbonylverbindung 0,0005 bis 500 kg/Stunde (h), insbesondere 0,005 bis 50 kg/h besonders bevorzugt 0,05 bis 50 kg/h Carbonylverbindung je Liter des sauren Festbettes beträgt. Als Volumen des sauren Festbettes wird dabei das gesamte vom sauren Festbett eingenommene Reaktorvolumen, inklusive der Hohlräume zwischen den Teilchen des sauren Festbettes betrachtet.

Das Reaktionsgemisch wird nach dem sauren Festbett mit dem Hydrierfestbett in Kontakt gebracht. Der Hydrierkatalysator ist ein Feststoff, es handelt sich daher um einen heterogenen Katalysator.

Das Hydrierfestbett enthält den festen Hydrierkatalysator oder besteht daraus. Neben dem Hydrierkatalysator kann das Hydrierfestbett weitere Bestandteile enthalten, zum Beispiel Inertmaterialen wie Füllstoffe.

In einer bevorzugten Ausführungsform besteht das Hydrierfestbett zu mindestens 50 Gew. %, insbesondere zu mindestens 80 Gew. %, besonderes bevorzugt zu mindestens 95 Gew % aus dem Hydrierkatalysator. In einer besonderen Ausführungsform besteht das Hydrierfestbett zu 100 Gew. % aus dem Hydrierkatalysator.

Bei dem Hydrierkatalysator kann es um einen üblichen heterogenen Hydrierkatalysator in Teilchenform handeln.

Heterogene Katalysatoren für die Hydrierung sind katalytisch aktive Elemente oder Verbindungen, sie können ohne Träger in Teilchenform vorliegen (Vollkontakt-Katalysatoren) oder sie können auf einen Träger, z. B. aus Calciumcarbonat, Siliciumoxid , Zirkoniumdioxid oder Aluminiumdioxid, aufgebracht sein (Trägerkatalysatoren).

Bevorzugte Hydrierkatalysatoren enthalten aktive Metalle, entweder in elementarer Form oder in Form von Verbindungen, z. B. Oxiden. Oft enthalten die Katalysatoren Mischungen von aktiven Metallen. Der Begriff Metall umfasst daher im Folgenden elementare Metalle oder auch Metalle, wie sie in chemischen Bindungen, sei es in ionischer Form oder kovalent gebundener Form vorliegen. Bei Verwendung der aktiven Metalle in Form ihrer Oxide oder gegebenenfalls auch sonstiger Verbindungen erfolgt im Allgemeinen bei höheren Temperaturen, insbesondere in Gegenwart von Wasserstoff, eine Reduktion der Oxide zu den Metallen. Dies kann mit Beginn der Umsetzung geschehen oder in einem separaten Schritt vorher durchgeführt werden.

Als Hydrierkatalysatoren kommen z.B. solche in Betracht, die ein Metall der Gruppen IVb, Vb, Vlb, Vllb, Vlllb, Ib, oder IIb enthalten.

In einer bevorzugten Ausführungsform handelt es sich bei dem Hydrierkatalysator um einen Trägerkatalysator.

Als Hydrierkatalysatoren kommen z. B. Katalysatoren in Betracht, die Nickel, Palladium, Platin, Cobalt, Rhodium, Iridium, Kupfer, Mangan, Zinn oder Ruthenium enthalten.

Bevorzugt sind Hydrierkatalysatoren, welche mindestens ein Metall der Cobalt-, Nickel- oder Kupfergruppe des Periodensystems enthalten, insbesondere mindestens ein Metall ausgewählt aus Cobalt, Rhodium, Iridium, Nickel, Palladium, Platin oder Kupfer. Insbesondere beträgt der Gehalt der vorstehenden Metalle der Cobalt-, Nickel- oder Kupfergruppe im Katalysator insgesamt mindestens 5 Gew. %, besonders bevorzugt mindestens 20 Gew. % und ganz besonders bevorzugt mindestens 50 Gew. %, bezogen auf die Gewichtssumme aller aktiven Metalle des Katalysators (bei Metallverbindungen, z.B. Oxiden, wird hier nur der Metallanteil berücksichtigt). In einer besonderen Ausführungsform werden Mischungen der vorstehenden Metalle verwendet. Weitere aktive Metalle, die mit den vorstehenden Metallen der Cobalt-, Nickel- oder Kupfergruppe mitverwendet werden können, sind z. B. Mangan, Zinn, Ruthenium oder auch Alkalimetalle und Erdalkalimetalle.

In einer besonders bevorzugten Ausführungsform enthalten die Hydrierkatalysatoren Cobalt, Nickel, Kupfer oder Mischungen dieser drei Metalle in Mengen von insgesamt 5 Gew.%, besonders bevorzugt mindestens 20 Gew.% und ganz besonders bevorzugt mindestens 50 Gew.%, bezogen auf die Gewichtssume aller aktiven Metalle des Hydrierkatalysators.

Die vorstehenden Metalle können elementar oder als Verbindung, z. B. als Oxide vorliegen. Als Katalysatoren, welche Metalle in ihrer elementaren Form enthalten seien Raney-Nickel oder Raney-Cobalt erwähnt.

Das Hydrierfestbett kann in dem Reaktor so angebracht sein, dass es in Strömungsrichtung des Reaktionsgemisches unmittelbar oder, je nach den apparativen Gegebenheiten, in einem definierten Abstand auf das saure Festbett folgt.

Das Hydrierfestbett kann in beliebiger Weise in den Reaktor eingebracht werden. Im Allgemeinen wird es so eingebracht, dass der gesamte Strom der Edukte durch das Hydrierfestbett geführt wird. Das Hydrierfestbett füllt daher im Allgemeinen den gesamten durchströmten Querschnitt des Reaktors aus. Das Volumen des Hydrierfestbettes wird vorzugsweise so gewählt, dass die Belastung des Hydrierfestbettes mit dem Massestrom der Carbonylverbindung 0,005 bis 50 kg/Stunde (h), insbesondere 0,05 bis 50 kg/h je Liter des Hydrierfestbettes beträgt. Als Volumen des Hydrierfestbettes wird dabei das gesamte vom Hydrierfestbett eingenommene Reaktorvolumen, inklusive der Hohlräume zwischen den Teilchen des Hydrierfestbettes betrachtet.
Das Volumen des Hydrierfestbettes kann größer oder kleiner als das Volumen des sauren Festbettes sein.

Das Volumenverhältnis des sauren Festbettes zum Hydrierfestbett kann zum Beispiel 0,05 bis 50, vorzugsweise 0,1 bis 10 betragen. Insbesondere können die Volumina des sauren Festbettes und Hydrierfestbettes gleich oder ähnlich sein, was einem Volumenverhältnis von 0,5 bis 2, bzw. 0,8 bis 1,25 entspricht.

Die Umsetzung der Carbonylverbindung mit dem sekundären Amin und Wasserstoff kann z. B. bei Temperaturen von 20 bis 200°C, vorzugsweise 40 bis 120°C, besonders bevorzugt 60 bis 100°C erfolgen. Die Umsetzung kann z. B. bei Normaldruck oder Überdruck durchgeführt werden. In einer bevorzugten Ausführungsform wird sie bei erhöhtem Druck z. B. von 1,1 bis 200 bar, insbesondere 5 bis 100 bar und ganz besonders bevorzugt 40 bis 100 bar durchgeführt. Der Druck wird vorzugsweise durch den entsprechenden Druck des zugeführten Wasserstoffs oder Gasgemisches (z. B. Wasserstoff und Inertgas), eingestellt.
Das Reaktionsprodukt wird nach Durchströmen des Hydrierfestbettes kontinuierlich aus der Reaktionszone entfernt.
Bei Verwendung von Wasser oder eines hydrophilen Lösemittel für das sekundäre Amin kann das Reaktionsprodukt zweiphasig sein. Zum Beispiel wird bei der Herstellung von 2-Chlordimethylbenzylamin aus den Verbindungen der Formel I und II unter Mitverwendung eines hydrophilen Lösemittels wird eine organische Phase erhalten, welche das 2- Chlordimethylbenzylamin und gegebenenfalls nicht umgesetztes 2-Chlorbenzaldehyd und gegebenenfalls Nebenprodukte enthält. Die wässrige, bzw. hydrophile Phase enthält das Lösemittel, in dem das sekundäre Amin gelöst war (Wasser bzw. ein hydrophiles Lösemittel), nicht umgesetztes sekundäres Amin und gegebenenfalls darin lösliche Nebenprodukte. Die organische Phase kann leicht abgetrennt werden. Die Reinigung oder Abtrennung der nicht umgesetzten Ausgangsstoffe (2-Chlorbenzaldehyd) kann z. B. durch Destillation oder Rektifikation erfolgen. Vorzugsweise wird bei der Umsetzung kein Lösemittel mitverwendet. Es wird daher als Reaktionsprodukt nur eine organische, homogene Phase erhalten, welche das Reaktionsprodukt, 2-Chlordialkylbenzylamin, gegebenenfalls nicht umgesetzte Ausgangsstoffe, 2-Chlorbenzaldehyd, und gegebenenfalls darin lösliche Nebenprodukte enthält. Das sekundäre Amin kann durch Destillation oder Rektifikation abgetrennt und, wenn gewünscht, in die Umsetzung zurückgeführt werden. Dimethylamin (Siedepunkt 7°C), ist bei entsprechenden Temperaturen flüchtig und kann besonders leicht bereits abgetrennt werden (Entgasung). Die organische Phase kann gegebenenfalls durch Destillation oder Rektifikation weiter aufgearbeitet bzw. gereinigt werden.

Durch das erfindungsgemäße Verfahren sind Produkte einer reduktiven Aminierung, 2-Chlordialkylbenzylamine, in einfacher und wirtschaftlicher Weise erhältlich. Das Verfahren ermöglicht die Herstellung von Aminen durch reduktive Aminierung in hoher Ausbeute und Selektivität.

### Beispiele

### Vergleichsbeispiel:

### Kontinuierliche Aminierung von 2-Chlorbenzaldehyd mit Dimethylamin ohne ZrO2-Schüttung

Ein beheizter Rohrreaktor mit einer Länge von 200 cm und einem Innendurchmesser von etwa 3,2 cm wurde mit einer Schicht Glaskugeln und anschließend 500 ml eines Nickel-, Cobalt- und Kupferhaltigen Katalysators geträgert auf ZrO2 befüllt. Abschließend wurde der Reaktor mit einer Schicht Glaskugeln aufgefüllt.

Vor der Reaktion wurde der Katalysator bei 280 °C unter Durchleiten von 200 l/h Wasserstoff bei Normaldruck für 24 h aktiviert. Nach Absenken der Temperatur auf 75 °C und Erhöhung des Druckes auf 80 bar wurden von unten nach oben pro Stunde 2-Chlorbenzaldehyd (100-300 g/h), Dimethylamin (320-350 g/h) und Wasserstoff (60-150 Normliter/h) dosiert. In regelmäßigen Abständen wurden Proben genommen und diese gaschromatographisch analysiert.

In weiteren Vergleichsbeispielen wurden die Mengen an 2-Chlorbenzaldehyd und Dimethylamin, die pro Zeiteinheit in den Reaktor geführt wurden, variiert (in der Tabelle kurz als "Belastung" in kg/Liter und Stunde). Auch wurden das Äquivalentverhältnis von Dimethylamin zu Chlorbenzaldehyd, der Druck und die Temperatur variiert (siehe Tabelle).

### Beispiel:

### Kontinuierliche Aminierung von 2-Chlorbenzaldehyd mit Dimethylamin mit ZrO2-Schüttung

In einem beheizten Rohrreaktor mit einer Länge von 200 cm und einem Innendurchmesser von etwa 3,2 cm wurde der untere Teil des Reaktors mit ca. 450 mL ZrO2 (3 mm Stränge) bestückt. Nach Einfüllen eines Nickel-, Cobalt- und Kupferhaltigen Katalysators geträgert auf ZrO2 (500 mL, 3x4 mm Tabletten) wurde das verbleibende Restvolumen des Rohrreaktors mit Glaskugeln aufgefüllt.

Vor der Reaktion wurde der Katalysator bei 280 °C unter Durchleiten von 200 l/h Wasserstoff bei Normaldruck für 24 h aktiviert. Nach Absenken der Temperatur auf 75 °C und Erhöhung des Druckes auf 80 bar wurden von unten nach oben pro Stunde 2-Chlorbenzaldehyd (100-300 g/h), Dimethylamin (320-350 g/h) und Wasserstoff (60-150 Nl/h) dosiert. In regelmäßigen Abständen wurden Proben genommen und diese gaschromatographisch analysiert.

In weiteren Beispielen wurden die Mengen an 2-Chlorbenzaldehyd und Dimethylamin, die pro Zeiteinheit in den Reaktor geführt wurden, variiert. Auch wurden wiederum das Äquivalentverhältnis von Dimethylamin zu Chlorbenzaldehyd, der Druck und die Temperatur variiert (siehe Tabelle).

**Tabelle:**

| Parameter und Ergebnisse zu den Vergleichsbeispielen und Beispielen | | | | | |
|---|---|---|---|---|---|
| | Vergleichsbeispiel | | | | |
| Belastung [kg/l*h] | 0,2 | 0,2 | 0,3 | 0,4 | 0,5 |
| Temp. [°C] | 60 | 70 | 60 | 60 | 70 |
| Äquiv. DMA (mol_{DMA}/mol_{oCIBA}) | 10 | 10 | 10 | 7 | 7 |
| Druck [bar] | 40 | 80 | 80 | 80 | 80 |
| Ausbeute ohne ZrO₂-Schüttung | 79,9 | 94,8 | 95,3 | 96,3 | 96,8 |

| | Beispiel | | | | |
|---|---|---|---|---|---|
| Belastung [kg/l*h] | 0,2 | 0,2 | 0,3 | 0,4 | 0,5 |
| Temp. [°C] | 60 | 70 | 75 | 75 | 75 |
| Äquiv. DMA (mol_{DMA}/mol_{oCIBA}) | 10 | 10 | 7,3 | 5,5 | 4,4 |
| Druck [bar] | 40 | 80 | 80 | 80 | 80 |
| Ausbeute mit ZrO₂-Schüttung | 94,7 | 97,7 | 98,0 | 98,7 | 98,1 |

## Patentansprüche

1. Verfahren zur reduktiven Aminierung durch Umsetzung einer Carbonylverbindung mit einem sekundären Amin und Wasserstoff, **dadurch gekennzeichnet, dass** die Umsetzung kontinuierlich durchgeführt wird und das Reaktionsgemisch, welches die Carbonylverbindung, das sekundäre Amin und Wasserstoff enthält, zunächst mit einem sauren Festbett und danach mit einem Festbett des Hydrierkatalysator (im Nachfolgenden Hydrierfestbett genannt) in Kontakt gebracht wird und es sich bei der Carbonylverbindung um ein 2-Chlorbenzaldehyd der Formel I 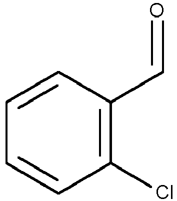 handelt, es sich bei dem sekundären Amin um ein Dialkylamin der Formel II 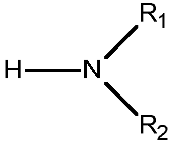 worin R1 und R2 jeweils unabhängig voneinander für eine C1- bis C10 Alkylgruppe stehen, handelt und es sich es sich um ein Verfahren zur Herstellung von 2-Chlordialkylbenzylamin der Formel III 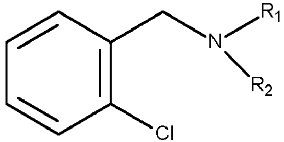 worin R₁ und R₂ die obige Bedeutung haben, handelt.

2. Verfahren gemäß Anspruch 1 , **dadurch gekennzeichnet, dass** das Molverhältnis der Carbonylverbindung zum sekundären Amin 1 : 1 bis 1: 20 beträgt

3. Verfahren gemäß einem der Ansprüche 1 oder 2 , **dadurch gekennzeichnet, dass** das saure Festbett Titandioxid, Zirkoniumdioxid, Aluminiumoxid, Siliciumdioxid, oder Mischoxide von Aluminium und Silicium (Zeolithe) enthält oder daraus besteht.

4. Verfahren gemäß einem der Ansprüche 1 bis 3, **dadurch gekennzeichnet, dass** das saure Festbett Zirkoniumdioxid enthält oder daraus besteht.

5. Verfahren gemäß einem der Ansprüche 1 bis 4, **dadurch gekennzeichnet, dass** der Hydrierkatalysator mindestens ein Metall enthält, welches ausgewählt ist aus Nickel, Palladium, Platin, Cobalt, Rhodium, Kupfer oder Iridium.

6. Verfahren gemäß einem der Ansprüche 1 bis 5, **dadurch gekennzeichnet, dass** es sich bei dem Hydrierkatalysator um einen Trägerkatalysator handelt.

7. Verfahren gemäß einem der Ansprüche 1 bis 6, **dadurch gekennzeichnet, dass** die Belastung des sauren Festbetts 0,0005 bis 500 kg/h Carbonylverbindung je Liter des sauren Festbetts beträgt..

8. Verfahren gemäß einem der Ansprüche 1 bis 7, **dadurch gekennzeichnet, dass** die Belastung des Hydrierfestbettes 0,005 bis 50 kg/h Carbonylverbindung je Liter des Hydrierfestbettes beträgt.

9. Verfahren gemäß einem der Ansprüche 1 bis 8, **dadurch gekennzeichnet, dass** das Volumenverhältnis des sauren Festbettes zum Hydrierfestbett 0,1 bis 10 beträgt.

## Claims

1. A process for effecting reductive amination by reacting a carbonyl compound with a secondary amine and hydrogen, wherein the reaction is carried out as a continuous operation and the reaction mixture comprising the carbonyl compound, the secondary amine and hydrogen is contacted first with an acidic fixed bed and then with a fixed bed of hydrogenation catalyst (hereinafter referred to as a hydrogenation fixed bed) and a 2-chlorobenzaldehyde of formula I 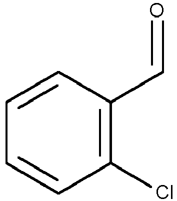 is used as the carbonyl compound, the secondary amine is a dialkylamine of formula II 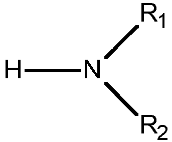 where R₁ and R₂ are each independently a C1 to C10 alkyl group and a process for preparing the 2-chlorodialkyl-benzylamine of formula III 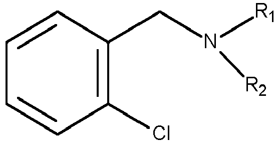 where both R₁ and R₂ are as defined above is used.

2. The process according to claim 1, wherein the molar ratio of the carbonyl compound to the secondary amine is from 1:1 to 1:20.

3. The process according to any one of claims 1 and 2, wherein the acidic fixed bed comprises or consists of titanium dioxide, zirconium dioxide, aluminum oxide, silicon dioxide or mixed oxides of aluminum and silicon (zeolites).

4. The process according to any one of claims 1 to 3, wherein the acidic fixed bed comprises or consists of zirconium dioxide.

5. The process according to any one of claims 1 to 4, wherein the hydrogenation catalyst comprises at least one metal selected from nickel, palladium, platinum, cobalt, rhodium, copper or iridium.

6. The process according to any one of claims 1 to 5, wherein the hydrogenation catalyst is a supported catalyst.

7. The process according to any one of claims 1 to 6, wherein the space velocity over the acidic fixed bed is from 0.0005 to 500 kg/h of carbonyl compound per liter of the acidic fixed bed.

8. The process according to any one of claims 1 to 7, wherein the space velocity over the hydrogenation fixed bed is from 0.005 to 50 kg/h of carbonyl compound per liter of hydrogenation fixed bed.

9. The process according to any one of claims 1 to 8, wherein the volume ratio of the acidic fixed bed to the hydrogenation fixed bed is in the range from 0.1 to 10.

## Revendications

1. Procédé d'amination réductrice par mise en réaction d'un composé de carbonyle avec une amine secondaire et de l'hydrogène, **caractérisé en ce que** la réaction est réalisée en continu et le mélange réactionnel qui contient le composé de carbonyle, l'amine secondaire et l'hydrogène est tout d'abord mis en contact avec un lit fixe acide, puis avec un lit fixe du catalyseur d'hydrogénation (par la suite nommé lit fixe d'hydrogénation), et le composé de carbonyle est un 2-chlorobenzaldéhyde de formule I 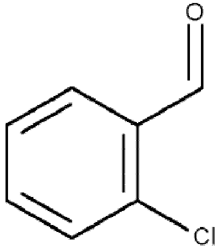 l'amine secondaire est une dialkylamine de formule II 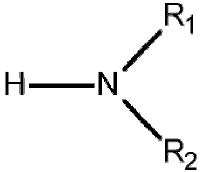 dans laquelle R₁ et R₂ représentent chacun indépendamment l'un de l'autre un groupe alkyle en C1 à C10, et il s'agit d'un procédé de fabrication d'une 2-chlorodialkylbenzylamine de formule III 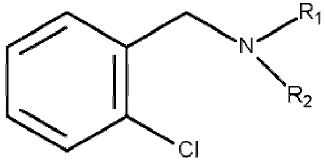 dans laquelle R₁ et R₂ ont la signification précédente.

2. Procédé selon la revendication 1, **caractérisé en ce que** le rapport molaire entre le composé de carbonyle et l'amine secondaire est de 1:1 à 1:20.

3. Procédé selon l'une quelconque des revendications 1 ou 2, **caractérisé en ce que** le lit fixe acide contient du dioxyde de titane, du dioxyde de zirconium, de l'oxyde d'aluminium, du dioxyde de silicium ou des oxydes mixtes d'aluminium et de silicium (zéolithe) ou en est constitué.

4. Procédé selon l'une quelconque des revendications 1 à 3, **caractérisé en ce que** le lit fixe acide contient du dioxyde de zirconium ou en est constitué.

5. Procédé selon l'une quelconque des revendications 1 à 4, **caractérisé en ce que** le catalyseur d'hydrogénation contient au moins un métal, qui est choisi parmi le nickel, le palladium, le platine, le cobalt, le rhodium, le cuivre ou l'iridium.

6. Procédé selon l'une quelconque des revendications 1 à 5, **caractérisé en ce que** le catalyseur d'hydrogénation est un catalyseur supporté.

7. Procédé selon l'une quelconque des revendications 1 à 6, **caractérisé en ce que** le chargement du lit fixe acide est de 0,0005 à 500 kg/h de composé de carbonyle par litre du lit fixe acide.

8. Procédé selon l'une quelconque des revendications 1 à 7, **caractérisé en ce que** le chargement du lit fixe d'hydrogénation est de 0,005 à 50 kg/h de composé de carbonyle par litre du lit fixe d'hydrogénation.

9. Procédé selon l'une quelconque des revendications 1 à 8, **caractérisé en ce que** le rapport volumique entre le lit fixe acide et le lit fixe d'hydrogénation est de 0,1 à 10.
